## Europäisches Patentamt
## European Patent Office
## Office européen des brevets

(11) Veröffentlichungsnummer : **0 032 989 B1**

(19)

---

(12) **EUROPÄISCHE PATENTSCHRIFT**

---

(45) Veröffentlichungstag der Patentschrift :
16.02.83

(51) Int. Cl.³ : **C 07 D301/16, C 07 D303/04**

(21) Anmeldenummer : **80107592.0**

(22) Anmeldetag : **04.12.80**

---

(54) **Verfahren zur Herstellung von alpha-Epoxiden mit 11 bis 24 Kohlenstoffatomen.**

---

(30) Priorität : **26.01.80 DE 3002826**

(43) Veröffentlichungstag der Anmeldung :
**05.08.81 Patentblatt 81/31**

(45) Bekanntmachung des Hinweises auf die Patenterteilung : **16.02.83 Patentblatt 83/07**

(84) Benannte Vertragsstaaten :
**AT BE CH FR GB IT LI NL SE**

(56) Entgegenhaltungen :
**DE A 1 568 016
DE A 1 618 625
US A 2 485 160
US A 2 774 774
Chemical Abstracts Band 41, Nr. 21, 10. November 1947 Columbus, Ohio, USA D. SWERN « Electronic interpretation of the reaction of olefins with organic per acids » Spalte 6870f**

(73) Patentinhaber : Degussa Aktiengesellschaft
**Weissfrauenstrasse 9
D-6000 Frankfurt am Main 1 (DE)**

**Henkel Kommanditgesellschaft auf Aktien
Postfach 1100 Henkelstrasse 67
D-4000 Düsseldorf 1 (DE).**

(72) Erfinder : Käbisch, Gerhard, Dr.
**Palmstrasse 1
D-7888 Rheinfelden 5 (DE)**
Erfinder : Trübe, Rudolf
**Ernst-Reuter-Strasse 22
D-7888 Rheinfelden (DE)**
Erfinder : Wittmann, Hans
**Katzenbuckelweg 12
D-7888 Rheinfelden (DE)**
Erfinder : Raupach, Siegfried
**Langentalstrasse 24
D-7888 Rheinfelden (DE)**
Erfinder : Malitius, Horst
**Blümleacker 5
D-7888 Rheinfelden (DE)**
Erfinder : Lindemann, Manfred, Dr. Dipl.-Chem.
**Eipass-Strasse 83
D-5650 Solingen 19 (DE)**

---

Jouve, 18, rue St-Denis, 75001 Paris, France

## Verfahren zur Herstellung von alpha-Epoxiden mit 11 bis 24 Kohlenstoffatomen

Die Erfindung betrifft ein Verfahren zur Herstellung von α-Epoxiden mit 11 bis 24 Kohlenstoffatomen durch Umsetzung von aliphatischen α-Olefinen mit 11 bis 24 Kohlenstoffatomen mit *in situ* aus Ameisensäure und Wasserstoffperoxid gebildeter Perameisensäure bei erhöhter Temperatur, unter Rühren des Reaktionsgemisches und unter Zudosierung des Wasserstoffperoxids.

Es ist bereits seit langem bekannt, die Ester höherer ungesättigter Fettsäuren mit innenständiger Doppelbindung, beispielsweise Sojabohnenöl, durch Umsetzung mit *in situ* aus Ameisensäure und Wasserstoffperoxid gebildeter Perameisensäure zu epoxidieren (vgl. z. B. US-PS 2 485 160).

Eine Übertragung der dabei gewonnenen Erkenntnisse auf die Epoxidierung von längerkettigen reinen Olefinen mit endständiger Doppelbindung, sogenannte α-Olefine, scheiterte an den nur schlechten Ausbeuten. Infolgedessen wurde in der Literatur übereinstimmend immer wieder darauf hingewiesen, dass α-Olefine mit *in situ*-Perameisensäure praktisch nicht epoxidierbar sind (vgl. z. B. Chemical Week vom 6. April 1963, Übersicht auf Seite 60).

Dies obwohl schon im Jahre 1947 in Chemical Abstracts, Band 41, Nr. 21, Spalte 6870 f, gesagt wurde, daß Perameisensäure rasch mit Olefinen reagiert, die eine oder zwei der Doppelbindung benachbarte Elektronen abstoßende Gruppen enthalten, obzwar bei Olefinen, die nur eine solche Gruppe enthalten, eine langsame Reaktion erwartet würde.

Aus der DE-OS 1 618 625 ist ein Verfahren zur Herstellung von Oxiranen durch Umsetzung einer eine $>C = C<$ -Gruppe enthaltenden Verbindung mit vorgefertigter Perameisensäure bekannt, bei dem die Umsetzung in einem hydrophoben organischen Lösungsmittel durchgeführt wird und das Reaktionsgemisch im wesentlichen frei von Mineralsäure und im wesentlichen wasserfrei ist.

Aus der US-PS 2 774 774 ist ein Verfahren zur Epoxydierung von olefinisch ungesättigten höheren Fettsäuren bekannt, bei dem diese Säuren mit einem inerten organischen Lösungsmittel und Ameisensäure versetzt werden und dann das Gemisch auf eine Temperatur zwischen etwa 50 und 80 °C erwärmt und mit wässrigem Wasserstoffperoxid versetzt wird. Das Reaktionsgemisch wird dabei nicht länger als etwa fünf Stunden auf einer Temperatur zwischen 50 und 80 °C gehalten. Die Ameisensäure wird in einer Menge zwischen 0,25 und 1,0 Mol und das Wasserstoffperoxid in einer Menge von etwa 1 Mol, jeweils pro Mol zu epoxydierender Doppelbindung, eingesetzt.

Schließlich wurde durch die DE-OS 1 568 016 ein Verfahren bekannt, bei dem α-Epoxide durch Umsetzung in einem mit Wasser nicht mischbaren Lösungsmittel mit einer *in situ* in einer wässrigen Phase gebildeten aliphatischen Persäure epoxidiert werden. Bei dem bekannten Verfahren muß das Reaktionsgemisch in einer besonders sanften Weise gerührt werden, so daß zwischen den beiden Phasen eine einzige Zwischenfläche aufrecht erhalten wird. Eine Dispergierung der beiden Phasen unter Tröpfchenbildung ist unbedingt zu vermeiden. Im allgemeinen wird ein Katalysator für die Bildung der aliphatischen Persäure, z. B. Schwefelsäure, mitverwendet. Obwohl auch andere aliphatische Säuren genannt werden, wird in allen Beispielen Essigsäure verwendet, weil diese besonders wirksam und besonders wirtschaftlich ist. Sie wird in Mengen von 0,5 bis 1,5 Mol pro Mol zu epoxidierender Doppelbindung eingesetzt. Obwohl das bekannte Verfahren infolge der Verwendung eines Lösungsmittels und der erforderlichen besonderen Vorsichtsmaßnahmen beim Rühren recht aufwendig ist, erfordert es zur Erzielung annehmbarer Ausbeuten sehr lange Reaktionszeiten von bis zu 28 Stunden.

Gegenstand der Erfindung ist nun ein Verfahren zur Herstellung von α-Epoxiden mit 11 bis 24 Kohlenstoffatomen durch Umsetzung von aliphatischen α-Olefinen mit 11 bis 24 Kohlenstoffatomen mit *in situ* aus Ameisensäure und Wasserstoffperoxid gebildeter Perameisensäure bei erhöhter Temperatur, unter Rühren des Reaktionsgemisches und unter Zudosierung des Wasserstoffperoxids, dadurch gekennzeichnet, daß man die Umsetzung in Abwesenheit von Lösungsmitteln und Katalysatoren bei einer Temperatur zwischen 40 und 90 °C vornimmt, die wässrige und die organische Phase durch Rühren bis zur Tropfenbildung vermischt, in der wässrigen Phase für einen Zeitraum von mindestens 5 Stunden eine über 5 Gewichtsprozent liegende Konzentration an Ameisensäure und eine zwischen 10 und 70 Gewichtsprozent liegende Konzentration an Wasserstoffperoxid aufrecht erhält, die Ameisensäure in einer Menge von weniger als 0,5 Mol und das Wasserstoffperoxid in einer Menge von mindestens 1,05 Mol, jeweils pro Mol zu epoxidierender Doppelbindung, einsetzt und das Wasserstoffperoxid während eines Zeitraumes von mindestens 3 Stunden zudosiert.

Durch das erfindungsgemäße Verfahren können die gewünschten α-Epoxide innerhalb relativ kurzer Reaktionszeiten in sehr hohen Ausbeuten erhalten werden. Es wäre grundsätzlich auch auf α-Olefine mit weniger als 11 oder mit mehr als 24 Kohlenstoffatomen anwendbar. Bei α-Olefinen mit weniger als 11 Kohlenstoffatomen sinkt jedoch infolge der Bildung von Nebenprodukten die Epoxidausbeute leicht auf unter 90 % liegende Werte ab. Andererseits sind reine α-Olefine mit über 24 Kohlenstoffatomen zwar nach dem erfindungsgemäßen Verfahren leicht epoxydierbar, sie bedingen jedoch wegen ihrer hohen Schmelzpunkte meist den zusätzlichen Einsatz von Lösungsmitteln.

Als Ausgangsmaterial dienen daher Olefine mit 11 bis 24 Kohlenstoffatomen, die eine endständige Doppelbindung aufweisen und zu einem 1,2-Epoxid umgesetzt werden. Solche Olefine sind beispielsweise 1-Hendecen, 1-Dodecen, 1-Hexadecen, 1-Octadecen oder 1-Eicosen. Ebensogut können auch Mischungen verschiedener α-Olefine eingesetzt werden. Besonders vorteilhaft ist das erfindungsgemäße Verfahren für die Herstellung von α-Epoxiden mit 14 bis 20 Kohlenstoffatomen aus den entsprechenden α-Olefinen.

Um die Temperatur des Reaktionsgemisches in dem Bereich zwischen 40 und 90 °C halten zu können, muß, insbesondere bei größeren Ansätzen, für Möglichkeiten zu einer ausreichenden Abführung der freiwerdenden Reaktionswärme gesorgt werden.

Während der Umsetzung werden die wässrige und die organische Phase durch Rühren derart vermischt, dass die Phasengrenzfläche bis zur Tropfenbildung überwunden wird, dass also sowohl in der wässrigen Phase Tröpfchen der organischen Phase verteilt sind als auch umgekehrt in der organischen Phase Tröpfchen der wässrigen Phase. Gleichwohl ist bei den niedrigeren Olefinen, insbesondere solchen mit 11 oder 12 Kohlenstoffatomen, eine vergleichsweise milde Rührung vorzuziehen, während die höheren Olefine, ab etwa 14 Kohlenstoffatomen, so kräftig mit der wässrigen Phase durchmischt werden können, dass praktisch eine Emulsion gebildet wird.

Beim erfindungsgemässen Verfahren muss in der wässrigen Phase für eine Zeitspanne von mindestens 5 Stunden, vorzugsweise während der gesamten Reaktionsdauer, eine über 5 Gewichtsprozent liegende Konzentration an Ameisensäure aufrecht erhalten werden. Dies kann erreicht werden, indem die gesamte Ameisensäure vor Beginn der Umsetzung vorgelegt wird, oder indem ein Teil der Ameisensäure vor Beginn der Umsetzung vorgelegt und der Rest während der Umsetzung zudosiert wird, oder indem die gesamte Ameisensäure während der Umsetzung zudosiert wird.

Welche der genannten drei Möglichkeiten gewählt wird, hängt in erster Linie von Sicherheitsüberlegungen ab. Bevorzugt wird die Ameisensäure allenfalls teilweise vorgelegt und dann während der Umsetzung derart zudosiert, dass ihre Konzentration in der wässrigen Phase zu Beginn der Umsetzung über 10 Gewichtsprozent, vorzugsweise zwischen 10 und 20 Gewichtsprozent, liegt. Mit fortschreitendem Umsatz sollte sich die Konzentration der Ameisensäure dann verringern und gegen Ende der Umsetzung der Grenze von 5 Gewichtsprozent nähern. Solange die Bedingung der Mindestkonzentration von 5 Gewichtsprozent eingehalten werden kann, was unter anderem auch von der Konzentration des eingesetzten Wasserstoffperoxids abhängt, kann Ameisensäure von beliebiger Konzentration eingesetzt werden. Zweckmässig ist die Verwendung von Ameisensäure mit einer Konzentration von mindestens 70 Gewichtsprozent. Bevorzugt werden die handelsüblichen Konzentrationen zwischen 85 und 100 Gewichtsprozent. Insgesamt wird die Ameisensäure in einer Menge von weniger als 0,5 Mol pro Mol zu epoxidierender Doppelbindung eingesetzt. Als Mindestmenge reichen 0,15 Mol pro Mol zu epoxidierender Doppelbindung aus. Bevorzugt werden 0,25 bis 0,40 Mol verwendet.

Während der gleichen Zeitspanne von mindestens 5 Stunden, innerhalb derer in der wässrigen Phase eine Ameisensäurekonzentration von mindestens 5 Gewichtsprozent aufrecht erhalten wird, muss in der wässrigen Phase auch eine zwischen 10 und 70 Gewichtsprozent, vorzugsweise zwischen 30 und 70 Gewichtsprozent, liegende Konzentration an Wasserstoffperoxid aufrecht erhalten werden. Vorzugsweise wird auch die Konzentration an Wasserstoffperoxid während der gesamten Reaktionsdauer in dem genannten Bereich gehalten. Die vorgeschriebene Konzentration an Wasserstoffperoxid wird dadurch aufrecht erhalten, dass man die Gesamtmenge des Wasserstoffperoxids von ausreichender Konzentration während eines Zeitraumes von mindestens 3 Stunden zudosiert. Zweckmässig ist die Verwendung eines Wasserstoffperoxids mit einer Konzentration von über 50 Gewichtsprozent, vorzugsweise von über 68 Gewichtsprozent.

Besonders bevorzugt wird Wasserstoffperoxid mit einer Konzentration zwischen 80 und 90 Gewichtsprozent, vor allem dann, wenn das erfindungsgemässe Verfahren in einer einzigen Stufe durchgeführt wird. Insgesamt wird das Wasserstoffperoxid in einer Menge von mindestens 1,05 Mol pro Mol zu epoxidierender Doppelbindung eingesetzt. Bevorzugt wird ein Überschuss an Wasserstoffperoxid angewandt und das Wasserstoffperoxid in einer Menge zwischen 1,2 und 2,5 Mol, insbesondere zwischen 1,3 und 1,9 Mol, pro Mol zu epoxidierender Doppelbindung eingesetzt. Da sich das nach beendeter Umsetzung in der wässrigen Phase vorliegende verdünnte Wasserstoffperoxid in an sich bekannter Weise zurückgewinnen lässt, beträgt der tatsächliche Verbrauch an Wasserstoffperoxid aber nur etwa 1,05 bis etwa 1,50 Mol pro Mol zu epoxidierender Doppelbindung.

Für die praktische Durchführung des erfindungsgemässen Verfahrens bieten sich die verschiedenartigsten verfahrenstechnischen Massnahmen an. So kann z. B. die Aufrechterhaltung der vorgeschriebenen Konzentrationen an Ameisensäure und Wasserstoffperoxid in der wässrigen Phase dadurch erleichtert werden, dass man einen der zudosierten Menge an Ameisensäure und Wasserstoffperoxid entsprechenden Teil der abgereicherten wässrigen Phase kontinuierlich über ein Phasentrenngefäss aus dem Reaktionsgemisch entfernt. Diese Operation kann solange fortgesetzt werden, bis in der organischen Phase der gewünschte Umsatz erreicht ist. Eine solche Verfahrensweise wäre hinsichtlich der organischen Phase einstufig, hinsichtlich der wässrigen Phase dagegen kontinuierlich. Eine andere Möglichkeit besteht darin, dass man einen Austausch der wässrigen Phase vornimmt, wenn infolge der zu stark abgesunkenen Konzentration an Wasserstoffperoxid die Reaktionsgeschwindigkeit unwirtschaftlich gering geworden ist. Auch eine solche Verfahrensweise wäre hinsichtlich der organischen Phase einstufig, hinsichtlich der wässrigen Phase jedoch zwei- oder mehrstufig. Als weitere Möglichkeit soll schliesslich noch eine Ausführungsform erwähnt werden, bei der man nach Art einer Gegenstromkaskade oder in einer Mixer-Settler-Batterie organische und wässrige Phase kontinuierlich derart gegeneinander führt, dass in den ersten Apparat das frische Olefin eindosiert und dort mit der am weitesten abgereicherten wässrigen Phase umgesetzt wird, während im letzten Apparat frische Ameisensäure und frisches hochkonzentriertes Wasserstoffperoxid mit der bereits weitgehend epoxidierten organischen Phase zur Umsetzung kommt. Zwischen den geschilderten verfahrenstechnischen Möglichkeiten gibt es zahlreiche Übergangsformen, die von einer chargenweisen Durchführung bis hin zur vollkontinuierlichen Gestaltung reichen.

Das erfindungsgemässe Verfahren ist auch in grosstechnischen Produktionsanlagen gefahrlos durchführbar, wenn einige sicherheitstechnische Massnahmen ergriffen werden. So können z. B. automatische Sicherheitsventile vorgesehen werden, die sich öffnen und den Ansatz mit Wasser verdünnen, wenn die Mischorgane ausfallen, die Menge des durch Zersetzung von Wasserstoffperoxid oder Perameisensäure gebildeten Abgases einen Grenzwert überschreitet oder die Temperatur merklich über 90 °C ansteigt.

Die Umsetzung zwischen dem Olefin und der *in situ* gebildeten Perameisensäure wird vorteilhafterweise drucklos durchgeführt. Sie erfordert im allgemeinen eine Reaktionszeit zwischen 5 und 20 Stunden, insbesondere zwischen 8 und 10 Stunden.

Nach beendeter Epoxidierungsreaktion wird in Normalfall die wässrige Phase von der organischen Epoxidphase getrennt. Zur weiteren Reinigung empfiehlt es sich, die organische Phase von darin noch gelösten Wasserstoffperoxid- und Ameisensäure-Anteilen zu befreien. Bevorzugt wird hierzu ein zweimal hintereinander vorgenommenes Auswaschen mit Wasser, wobei das Volumenverhältnis zwischen der organischen Phase und dem Waschwasser jeweils etwa 10 : 1 beträgt. Wegen der geringen Wasserlöslichkeit der höheren $\alpha$-Epoxide kann das Auswaschen auch bei erhöhten Temperaturen vorgenommen werden. Die organische Phase muss daher nach beendeter Epoxidierungsreaktion nicht erst abgekühlt werden. Das nach dem letzten Auswaschen in der organischen Phase noch gelöste Wasser kann leicht in an sich bekannter Weise, z. B. durch leichtes Erwärmen unter vermindertem Druck, entfernt werden.

Bei der Herstellung von $\alpha$-Epoxiden aus einheitlichen kürzerkettigen $\alpha$-Olefinen mit 11 oder 12 Kohlenstoffatomen kann es unter Umständen vorteilhafter sein, die Epoxidierungsreaktion nicht bis zu nahezu vollständigem Umsatz fortzuführen, weil dann möglicherweise bereits die Bildung von Nebenprodukten einsetzt. Die Epoxidierungsreaktion kann beispielsweise nach einem zwischen 60 und 90 % liegenden Umsatz abgebrochen werden. Dann kann das leichter siedende $\alpha$-Olefin destillativ von dem höher siedenden $\alpha$-Epoxid abgetrennt und in Folgeansätzen mitverwendet werden. Auf diese Weise kann in jedem Fall ein hoher Umsatz des $\alpha$-Olefins und gleichzeitig eine hohe Epoxid-Ausbeute erhalten werden.

Beim Einsatz von Olefingemischen oder von einheitlichen längerkettigen $\alpha$-Olefinen mit mehr als 12 Kohlenstoffatomen werden auch ohne besondere Massnahmen hohe Umsätze und hohe Epoxidausbeuten erreicht. So gelingt beispielsweise die Epoxidierung von $\alpha$-$C_{14}$-Olefin bereits unter einem Olefinumsatz von etwa 96 % mit einer Ausbeute von 96 %. Bei der Epoxidierung von $\alpha$-$C_{18}$-Olefin liegt unter einem Olefinumsatz von etwa 96 % die Ausbeute bei 99 %.

Die nach dem erfindungsgemässen Verfahren hergestellten Epoxide sind so rein, dass sie für die meisten Verwendungszwecke direkt eingesetzt werden können. Sollte dies in speziellen Fällen erwünscht sein, können sie aber natürlich auch in an sich bekannter Weise, z. B. durch Totaldestillation unter vermindertem Druck, weiter gereinigt werden.

Die nach der Abtrennung der organischen Phase hinterbleibende abgereicherte wässrige Phase kann in an sich bekannter Weise zur Rückgewinnung des enthaltenen Wasserstoffperoxids aufgearbeitet werden.

Das erfindungsgemässe Verfahren soll durch die nachfolgenden Beispiele näher erläutert werden :

Beispiel 1

Als Ausgangsmaterial diente ein handelsüblicher Olefinschnitt, bestehend aus $\alpha$-$C_{12}$- und $\alpha$-$C_{14}$-Olefin (140 Mol = 32 l). Die Reaktionsapparatur (Inhalt 50 l) bestand aus einem Rührbehälter mit Phasentrennvorrichtung am unteren Auslauf, Einsatzwärmeaustauscher, Dosiergefässen für Wasserstoffperoxid und Ameisensäure mit dazugehörigen Dosierpumpen, Abgasmessvorrichtung und Thermometer. Die Temperatur wurde während des gesamten Versuchs auf 60 °C gehalten.

Zunächst wurden im Verlauf von 2 Stunden 84,0 Mol (= 2,67 l) eines 80 gewichtsprozentigen Wasserstoffperoxids und 17,5 Mol (= 0,70 l) einer 98 gewichtsprozentigen Ameisensäure eindosiert. Nach beendeter Zudosierung wurde noch 1,5 Stunden lang nachgerührt. Zu diesem Zeitpunkt lag in der wässrigen Phase das Wasserstoffperoxid in einer Konzentration von 38 Gewichtsprozent vor.

Nun begann hinsichtlich der wässrigen Phase die kontinuierliche Fahrweise, das heisst, es wurden stetig eindosiert 80 gewichtsprozentiges Wasserstoffperoxid und 98 gewichtsprozentige Ameisensäure und aus dem Phasentrenngefäss wurde gleichzeitig ein entsprechender Teil der wässrigen Phase abgelassen. Die Zusammensetzung der abgelassenen wässrigen Phase schwankte zwischen 38 und 40 Gewichtsprozent Wasserstoffperoxid, 1,5 bis 3,5 Gewichtsprozent Perameisensäure, 8 bis 12 Gewichtsprozent Ameisensäure, Rest Wasser.

Nach einer Gesamtreaktionszeit von (2,0 + 1,5 + 8,5 Stunden =) 12 Stunden wurde in der organischen Phase ein Epoxidgehalt von 91,8 Gewichtsprozent und ein Olefinrestgehalt von 3,9 Gewichtsprozent ermittelt. Die Reaktion wurde abgebrochen und nach Phasentrennung wurde die organische Phase zweimal hintereinander mit je 3 l Wasser gewaschen.

Insgesamt kamen zum Einsatz 300 Mol (= 9,5 l) 80 gewichtsprozentiges Wasserstoffperoxid und 63 Mol (= 2,42 l) 98 gewichtsprozentige Ameisensäure. Die abgelassene wässrige Phase betrug insgesamt 10,6 l.

Beispiel 2

Als Ausgangsmaterial dienten 4 000 Mol (= 1 300 l) α-$C_{18}$-Olefin. Die Apparatur bestand aus einem emaillierten 2 m³-Rührkessel mit den im Beispiel 1 genannten Zusatzeinrichtungen. Aus Gründen der Sicherheit war ein wassergefüllter Hochbehälter mit automatischem Schnellschlussventil angeschlossen. Die Reaktionswärme wurde über aussenliegende Kühler abgeführt, über die stetig ein Teil des Reaktionsgemisches umgepumpt wurde. Die Epoxidierung erfolgte bei 60 °C in drei Stufen bei einer Gesamtreaktionszeit von 10 Stunden.

In der ersten Stufe, Dauer 3,5 Stunden, kamen zum Einsatz 78,5 l 88 gewichtsprozentiges Wasserstoffperoxid und 19,5 l 98 gewichtsprozentige Ameisensäure, von der 5 l vorgelegt wurden. Das Wasserstoffperoxid und die restliche Ameisensäure wurden im Verlauf von 2 Stunden eindosiert. Nach beendeter Zudosierung wurde der Ansatz noch 1,5 Stunden lang weitergerührt. Zu diesem Zeitpunkt enthielt die wässrige Phase 34,5 Gewichtsprozent Wasserstoffperoxid, 8,5 Gewichtsprozent Ameisensäure und 2,1 Gewichtsprozent Perameisensäure. Sie wurde abgelassen. Der Epoxidgehalt in der organischen Phase betrug 42,5 Gewichtsprozent.

Die zweite Stufe wurde in der gleichen Weise durchgeführt wie die erste. Danach war der Epoxidgehalt auf 79,0 Gewichtsprozent angestiegen. Die abgelassene wässrige Phase enthielt 36,1 Gewichtsprozent Wasserstoffperoxid, 8,2 Gewichtsprozent Ameisensäure und 2,0 Gewichtsprozent Perameisensäure.

In der dritten Stufe, Dauer 3,0 Stunden, wurden im Verlauf einer Stunde eindosiert 33,5 l 88 gewichtsprozentiges Wasserstoffperoxid und 15,5 l 98 gewichtsprozentige Ameisensäure.

Nach einer Nachreaktionszeit von 2 Stunden enthielt die abgelassene wässrige Phase 36,1 Gewichtsprozent Wasserstoffperoxid, 7,5 Gewichtsprozent Ameisensäure und 2,2 Gewichtsprozent Perameisensäure.

Die nach Durchführung der dritten Stufe zurückgebliebene organische Phase wurde zweimal hintereinander mit je 100 l Wasser gewaschen und anschliessend eine halbe Stunde lang bei 70 Torr getrocknet. Sie wies einen Epoxidgehalt von 95,8 Gewichtsprozent und einen Olefingehalt von 3,2 Gewichtsprozent auf.

Beispiel 3

In einem 1 Liter-Mehrhalskolben, ausgestattet mit Rührer, zwei Tropftrichtern, Thermometer und einem Rückflusskühler mit dahintergeschalteter Gasuhr, wurden 2 Mol (= 336 g) α-$C_{12}$-Olefin auf 70 °C erwärmt und durch ein thermostatisiertes Bad bei dieser Temperatur gehalten. Unter Rühren wurden im Verlauf von 4 Stunden aus dem ersten Tropftrichter 0,4 Mol (= 18,8 g) einer 98 gewichtsprozentigen Ameisensäure und aus dem zweiten Tropftrichter 2,6 Mol (= 104 g) eines 85 gewichtsprozentigen Wasserstoffperoxids zudosiert. Nach beendeter Zudosierung wurde der Ansatz unter Rühren 2 weitere Stunden bei 70 °C gehalten. Dann wurden die beiden Phasen getrennt.

Die wässrige Phase enthielt ca. 30 Gewichtsprozent Wasserstoffperoxid, 1,5 Gewichtsprozent Perameisensäure und 5,5 Gewichtsprozent Ameisensäure. Die organische Phase enthielt 78,8 Gewichtsprozent Epoxid und 17,2 Gewichtsprozent Olefin.

Die organische Phase wurde zweimal hintereinander mit je 30 ml Wasser gewaschen und danach einer Teildestillation bei vermindertem Druck unterworfen, die bei einer Kopftemperatur von 122 °C (12 Torr) abgebrochen wurde. Das Destillat, insgesamt 64 g, enthielt 96 Gewichtsprozent Olefin und 4 Gewichtsprozent Epoxid und wurde in Folgeansätzen mitverwendet. Im Sumpfprodukt, insgesamt 298 g, wurde ein Epoxidgehalt von 94,7 Gewichtsprozent festgestellt. Durch anschliessende Feinfraktionierung (bei 12 Torr) wurde als Fraktion 2 ein 99,5 gewichtsprozentiges Epoxid erhalten:

Fraktion 1 : 120 – 124 °C = 16 g
Fraktion 2 : 124 – 125 °C = 264 g
Rückstand : > 125 °C = 18 g

Beispiel 4

410 l (= 1 412 Mol) α-$C_{16}$-Olefin wurden in einem heiz- und kühlbaren Edelstahl-Rührbehälter auf 70 °C erhitzt. Unter Kühlung wurde der Ansatz danach bei dieser Temperatur gehalten. In der Reaktionsstufe 1 wurden 1,4 l Ameisensäure (98 Gewichtsprozent) sofort zugegeben und eine weitere Menge von 12,2 l im Verlauf von 4 Stunden gleichmässig eindosiert. Im Verlauf von 3 Stunden erfolgte die Zudosierung einer Menge von 51,4 l Wasserstoffperoxid (88 Gewichtsprozent). Nach einer Gesamtreaktionszeit von 5 Stunden war die erste Reaktionsstufe beendet und es wurde eine Phasentrennung vorgenommen. Die wässrige Phase (= 51 l) wurde

abgelassen; sie enthielt 33,6 Gewichtsprozent Wasserstoffperoxid, 0,8 Gewichtsprozent Perameisensäure und 5,7 Gewichtsprozent Ameisensäure. Die im Rührkessel verbleibende organische Phase enthält 70,4 Gewichtsprozent $\alpha$-$C_{16}$-Epoxid und 28,8 Gewichtsprozent $\alpha$-$C_{16}$-Olefin. Unmittelbar nach der Phasentrennung wurde die Reaktionsstufe 2 durchgeführt. 0,5 l Ameisensäure wurden vorgelegt und 4,3 l im Verlauf von 3 Stunden eindosiert. In der 3-stündigen Eindosierungszeit wurden auch 20 l Wasserstoffperoxid bei guter Durchmischung eindosiert. Nach einer Gesamtreaktionszeit von wiederum 5 Stunden wurde auch die 2. Reaktionsstufe abgebrochen. Die abgelassene wässrige Phase (= 18 l) enthält 37,2 Gewichtsprozent Wasserstoffperoxid, 0,4 Gewichtsprozent Perameisensäure und 4,3 Gewichtsprozent Ameisensäure.

Die im Kessel verbleibende organische Phase wurde zweimal hintereinander mit je 30 l Wasser (bei 70 °C) gewaschen und nach der Waschwasserentfernung von geringen Mengen gelösten Wassers durch Anlegen von Vakuum (70 Torr) befreit. Die Analyse der so getrockneten organischen Phase ergab einen Epoxidgehalt von 93,9 Gewichtsprozent und einen Restolefingehalt von 4,1 Gewichtsprozent.

Beispiel 5

95 kg (404 Mol) eines Gemisches aus $\alpha$-Olefinen und Vinyliden-olefinen der Kohlenstoffzahl $C_{16}$-$C_{18}$ (Vinylidenanteil ca. 25 %; Jodzahl 108) und 3,95 kg (73 Mol) 85-gewichtsprozentige Ameisensäure wurden in einem mit Heiz- und Kühleinrichtung versehenen Rührgefäss auf 50 °C erwärmt. Im Verlauf von 90 Minuten wurden 16,6 kg (390,5 Mol) 80-gewichtsprozentiges Wasserstoffperoxid langsam zugegeben, wobei eine Temperatur von 60 °C aufrechterhalten wurde. Nach dem Ende der Wasserstoffperoxidzugabe wurde 6 1/2 Stunden bei 60 °C weitergerührt.

Nach dem Abkühlen wurde die wässrige Phase abgetrennt und verworfen. Zur organischen Phase wurden 2,6 kg (48 Mol) 85-gewichtsprozentige Ameisensäure gegeben. Nach dem Erwärmen des Gemisches auf 50 °C wurden im Verlauf von 90 Minuten 11,1 kg (261 Mol) 80-gewichtsprozentiges Wasserstoffperoxid zugegeben, wobei die Reaktionstemperatur wieder auf 60 °C einreguliert wurde. Anschliessend wurde die Mischung 6 1/2 Stunden bei 60 °C weitergerührt.

Aus dem abgekühlten Reaktionsgemisch wurde die organische Phase abgetrennt, zweimal mit heissem Wasser bis zum pH-Wert 4 gewaschen, danach mit 12 l 2-gewichtsprozentiger Natronlauge behandelt. Nach erneutem Waschen mit heissem Wasser wurde das Reaktionsprodukt durch Erhitzen auf 90 °C im Ringpumpenvakuum bei 30 Torr getrocknet und schliesslich über Filterhilfe filtriert.

Es wurden 100 kg farbloses Epoxyalkangemisch mit 5,83 Gewichtsprozent Epoxidsauerstoffgehalt (91,5 % des theoretischen Gehaltes) und der Jodzahl 3,72 erhalten.

Beispiel 6

95 kg (404 Mol) eines Gemisches aus $\alpha$-Olefinen und Vinylidenolefinen der Kohlenstoffzahl $C_{16}$-$C_{18}$ (Vinylidenanteil ca. 25 %; Jodzahl 108) und 3,27 kg (60 Mol) 85-gewichtsprozentige Ameisensäure wurden unter Rühren auf 50 °C erwärmt. Innerhalb einer Stunde wurden dann 14,7 kg (302,5 Mol) 70-gewichtsprozentiges Wasserstoffperoxid zugegeben, wobei die Temperatur der Mischung auf 60 °C gehalten wurde. Anschliessend wurde 6 Stunden lang bei 60 °C weitergerührt.

Nach Abschalten des Rührwerks und Phasentrennung wurde die wässrige Phase abgezogen und verworfen. Zur organischen Phase wurden 1,63 kg (30 Mol) 85-gewichtsprozentige Ameisensäure zugegeben. Nach dem Aufheizen der Mischung wurden bei 60 °C im Verlauf von einer Stunde 7,35 kg (151 Mol) 70-gewichtsprozentiges Wasserstoffperoxid zugegeben. Anschliessend wurde das Gemisch 4 Stunden lang bei 60 °C weitergerührt. Danach wurde die wässrige Phase abgetrennt und der Vorgang der zweiten Stufe wiederholt.

Die Aufarbeitung des Reaktionsgemisches analog Beispiel 5 ergab 100 kg Epoxyalkangemisch mit 5,81 Gewichtsprozent Epoxidsauerstoffgehalt (91,2 % des theoretischen Gehaltes) und der Jodzahl 4,4.

Beispiel 7

78,7 kg (400 Mol) Tetradecen-1 (Vinylidenanteil unter 1 %) und 4 kg (74 Mol) 85-gewichtsprozentige Ameisensäure wurden unter Rühren auf 50 °C erwärmt. Im Verlauf von 90 Minuten wurden anschliessend 15,3 kg (360 Mol) 80-gewichtsprozentiges Wasserstoffperoxid langsam zugegeben, wobei die Temperatur auf 60 °C gehalten wurde. Danach wurde das Gemisch 8 Stunden bei 60 °C weitergerührt.

Die wässrige Phase wurde abgelassen. Die organische Phase wurde mit 2 kg (37 Mol) 85-gewichtsprozentiger Ameisensäure versetzt. Nach dem Aufheizen der Mischung wurden bei 60 °C im Verlauf von 90 Minuten 10,2 kg (240 Mol) 80-gewichtsprozentiges Wasserstoffperoxid zugegeben und anschliessend das Gemisch 8 Stunden bei 60 °C weitergerührt.

Die Aufarbeitung des Reaktionsgemisches analog Beispiel 5 ergab 83 kg wasserhelles Tetradecenoxid mit 7,1 Gewichtsprozent Epoxidsauerstoffgehalt (94,4 % des theoretischen Gehaltes) und der Jodzahl 6,17.

Beispiel 8

78,7 kg (400 Mol) Tetradecen-1 wurden analog Beispiel 6 in 3 Reaktionsstufen mit insgesamt .6 kg (111 Mol) 85-gewichtsprozentiger Ameisensäure und 29 kg (597 Mol) 70-gewichtsprozentigem Wasserstoffperoxid bei 65 °C umgesetzt. Dabei wurden von den Gesamtmengen an Ameisensäure und Wasserstoffperoxid in der 1. Stufe 50 % und in der 2. und 3. Stufe jeweils 25 % eingesetzt. Die Gesamtreaktionszeit betrug in der 1. Stufe 8, in der 2. und 3. Stufe jeweils 6 Stunden.

Die Aufarbeitung des Reaktionsgemisches ergab 82,5 kg Tetradecenoxid mit 7,24 Gewichtsprozent Epoxidsauerstoff (96,3 % des theoretischen Gehaltes) und der Jodzahl 3,85.

### Ansprüche

1. Verfahren zur Herstellung von α-Epoxiden mit 11 bis 24 Kohlenstoffatomen durch Umsetzung von aliphatischen α-Olefinen mit 11 bis 24 Kohlenstoffatomen mit in situ aus Ameisensäure und Wasserstoffperoxid gebildeter Perameisensäure bei erhöhter Temperatur, unter Rühren des Reaktionsgemisches und unter Zudosierung des Wasserstoffperoxides, dadurch gekennzeichnet, daß man die Umsetzung in Abwesenheit von Lösungsmitteln und Katalysatoren bei einer Temperatur zwischen 40 und 90 °C vornimmt, die wässrige und die organische Phase durch Rühren bis zur Tropfenbildung vermischt, in der wässrigen Phase für einen Zeitraum von mindestens 5 Stunden eine über 5 Gewichtsprozent liegende Konzentration an Ameisensäure und eine zwischen 10 und 70 Gewichtsprozent liegende Konzentration an Wasserstoffperoxid aufrecht erhält, die Ameisensäure in einer Menge von 0,15 bis 0,5 Mol und das Wasserstoffperoxid in einer Menge von mindestens 1,05 Mol, jeweils pro Mol zu epoxidierender Doppelbindung, einsetzt und das Wasserstoffperoxid während eines Zeitraumes von mindestens 3 Stunden zudosiert.

2. Verfahren nach Anspruch 1, dadurch gekennzeichnet, dass man die Konzentration der Ameisensäure in der wässrigen Phase von über 5 Gewichtsprozent während der gesamten Reaktionsdauer aufrecht erhält.

3. Verfahren nach Anspruch 2, dadurch gekennzeichnet, dass man zu Beginn der Umsetzung die Konzentration der Ameisensäure in der wässrigen Phase auf über 10 Gewichtsprozent einstellt und sie sich im Verlaufe der Umsetzung der Grenze von 5 Gewichtsprozent gegen Ende der Umsetzung nähert.

4. Verfahren nach einem der Ansprüche 1 bis 3, dadurch gekennzeichnet, dass man eine Ameisensäure mit einer Konzentration von mindestens 70 Gewichtsprozent, vorzugsweise von 85 bis 100 Gewichtsprozent, einsetzt.

5. Verfahren nach einem der Ansprüche 1 bis 4, dadurch gekennzeichnet, dass man die Konzentration des Wasserstoffperoxids in der wässrigen Phase während der gesamten Reaktionsdauer in dem Bereich zwischen 10 und 70 Gewichtsprozent hält.

6. Verfahren nach einem der Ansprüche 1 bis 5, dadurch gekennzeichnet, dass man ein Wasserstoffperoxid mit einer Konzentration von mindestens 50 Gewichtsprozent, vorzugsweise von 80 bis 90 Gewichtsprozent, einsetzt.

7. Verfahren nach einem der Ansprüche 1 bis 6, dadurch gekennzeichnet, dass man das Wasserstoffperoxid in einer Menge zwischen 1,2 und 2,5 Mol, insbesondere zwischen 1,3 und 1,9 Mol, pro Mol zu epoxidierender Doppelbindung einsetzt.

### Claims

1. A process for the production of α-epoxides having from 11 to 24 carbon atoms by the reaction of aliphatic α-olefins having from 11 to 24 carbon atoms with performic acid which has formed in situ from formic acid and hydrogen peroxide, under elevated temperature, with stirring of the reaction mixture and with the metered addition of hydrogen peroxide, characterised in that the reaction is carried out at a temperature of from 40 to 90 °C in the absence of solvents and catalysts, the aqueous phase and the organic phase are mixed by stirring until drops form, a concentration of more than 5 % by weight of formic acid and a concentration of from 10 to 70 % by weight of hydrogen peroxide is maintained in the aqueous phase for a period of a least 5 hours, the formic acid is used in a quantity of from 0.15 to 0.5 mol and the hydrogen peroxide is used in a quantity of at least 1.05 mol, in each case per mol of double bond to be epoxidized, and the hydrogen peroxide is metered in over a period of at least 3 hours.

2. A process according to claim 1, characterised in that the concentration of formic acid in the aqueous phase of more than 5 % by weight is maintained for the complete duration of the reaction.

3. A process according to claim 2, characterised in that at the start of the reaction, the concentration of formic acid in the aqueous phase is adjusted to more than 10 % by weight and, during the course of the reaction, it approaches the limit of 5 % by weight towards the end of the reaction.

4. A process according to one of claims 1 to 3, characterised in that a formic acid is used which has a concentration of at least 70 % by weight, preferably from 85 to 100 % by weight.

5. A process according to one of claims 1 to 4, characterised in that the concentration of hydrogen peroxide in the aqueous phase is maintained in a range of from 10 to 70 % by weight for the complete duration of the reaction.

6. A process according to one of claims 1 to 5, characterised in that a hydrogen peroxide is used which has a concentration of at least 50 % by weight, preferably from 80 to 90 % by weight.

7. A process according to one of claims 1 to 6, characterised in that the hydrogen peroxide is used in a quantity of from 1.2 to 2.5 mols, in particular from 1.3 to 1.9 mol per mol of double bond to be epoxidized.

### Revendications

1. Procédé de fabrication d'α-époxydes avec 11 à 24 atomes de carbone, par réaction d'α-

oléfines aliphatiques à 11 à 24 atomes de carbone avec de l'acide performique formé *in situ* à partir d'acide formique et d'eau oxygénée, à température élevée, sous agitation du mélange réactionnel et en introduisant l'eau oxygénée, procédé caractérisé en ce que l'on conduit la réaction en l'absence de solvant et de catalyseur à une température comprise entre 40 à 90 °C, on mélange les phases aqueuse et organique par agitation jusqu'à la formation de gouttes, on maintient dans la phase aqueuse, pendant au moins cinq heures, une concentration en acide formique supérieure à 5 % en poids et une concentration en eau oxygénée comprise entre 10 et 70 % en poids, on met en œuvre l'acide formique dans une proportion de 0,15 à 0,5 mole et l'eau oxygénée dans une proportion d'au moins 1,05 mole, chaque fois par mole de liaison double à époxyder, et l'on introduit l'eau oxygénée en l'espace d'au moins trois heures.

2. Procédé selon la revendication 1, caractérisé en ce que la concentration en acide formique dans la phase aqueuse est maintenue à un niveau supérieur à 5 % en poids pendant toute la durée de la réaction.

3. Procédé selon la revendication 2, caractérisé en ce qu'au début de la réaction, on établit la concentration en acide formique dans la phase aqueuse à un niveau supérieur à 10 % en poids, et au cours de la réaction, cette concentration varie de façon à se rapprocher de la limite de 5 % en poids à la fin de la réaction.

4. Procédé selon l'une quelconque des revendications 1 à 3, caractérisé en ce que l'on met en œuvre de l'acide formique à une concentration d'au moins 70 % en poids, de préférence de 85 à 100 % en poids.

5. Procédé selon l'une quelconque des revendications 1 à 4, caractérisé en ce que l'on maintient la concentration de l'eau oxygénée dans la phase aqueuse pendant toute la durée de la réaction, dans une zone comprise entre 10 et 70 % en poids.

6. Procédé selon l'une quelconque des revendications 1 à 5, caractérisé en ce que l'on met en œuvre l'eau oxygénée à une concentration d'au moins 50 % en poids, de préférence 80 à 90 % en poids.

7. Procédé selon l'une quelconque des revendications 1 à 6, caractérisé en ce que l'on met en œuvre l'eau oxygénée dans une proportion comprise entre 1,2 et 2,5 moles, de préférence entre 1,3 et 1,9 mole, par mole de double liaison à époxyder.